Europäisches Patentamt

European Patent Office

Office européen des brevets

(11) Numéro de publication: **0 134 745**
**A1**

## (12) DEMANDE DE BREVET EUROPEEN

(21) Numéro de dépôt: **84420147.5**

(22) Date de dépôt: **31.08.84**

(51) Int. Cl.⁴: **A 61 M 5/00**

(30) Priorité: **02.09.83 FR 8314344**

(43) Date de publication de la demande:
**20.03.85 Bulletin 85/12**

(84) Etats contractants désignés:
**AT BE CH DE GB IT LI LU NL SE**

(71) Demandeur: **Meriaux, Henri**
**Les Résidences du Port de Mandelieu Avenue Henri**
**Clews La Napoule**
**F-06210 Mandelieu(FR)**

(72) Inventeur: **Meriaux, Henri**
**Les Résidences du Port de Mandelieu Avenue Henri**
**Clews La Napoule**
**F-06210 Mandelieu(FR)**

(74) Mandataire: **Maureau, Philippe et al,**
**Cabinet Germain & Maureau Le Britannia - Tour C 20, bld**
**Eugène Déruelle Boîte Postale 3011**
**F-69392 Lyon Cédex 03(FR)**

(54) **Dispositif de perfusion.**

(57) Ce dispositif est constitué par une capsule (2) réalisée en un matériau biocompatible, destinée à être implanté sous la peau du patient, comprenant un fond (3) dans lequel débouche une extrémité d'un cathéter (12), dont l'autre extrémité débouche dans une veine ou autre organe à perfuser, et un couvercle (4) qui, fixé sur le fond et tourné du côté de la peau du patient, présente une ouverture centrale (22) et sert au maintien en compression contre le fond d'une pastille auto-obturante (20), de part et d'autre de laquelle sont situées, d'une part, l'ouverture (22) ménagée dans le couvercle et, d'autre part, une cavité (8) ménagée dans le fond de la capsule et dans laquelle débouche le cathéter (12).

Application aux injections intraveineuses à cadence répétée.

FIG_4.

EP 0 134 745 A1

# DISPOSITIF DE PERFUSION

La présente invention a pour objet un dispositif de perfusion.

La perfusion de liquides médicaux est le plus souvent effectué par voie intraveineuse . Si une telle solution ne présente d'inconvénients ni pour le patient, ni pour le praticien lorsqu'elle est effectuée occasionnellement, il n'en est pas de même lorsqu'elle est répétée, comme tel est le cas lors d'un traitement de longue durée par exemple chez des malades souffrant d'un cancer. En effet, une répétition d'injections se traduit par un durcissement des vaisseaux dans les zones où sont pratiquées les piqures.

La présente invention vise à remédier à ces inconvénients en fournissant un dispositif de perfusion particulièrement bien adapté à des traitements de longue durée.

A cet effet, le dispositif qu'elle concerne est constitué par une capsule réalisée en un matériau biocompatible, destinée à être implantée sous la peau du patient, comprenant un fond dans lequel débouche une extrémité d'un cathéter, dont l'autre extrémité débouche dans une veine ou autre organe à perfuser, et un couvercle qui, fixé sur le fond et tourné du côté de la peau du patient, présente une ouverture centrale et sert au maintien en compression contre le fond d'une pastille auto-obturante, de part et d'autre de laquelle sont situées, d'une part, l'ouverture ménagée dans le couvercle et, d'autre part, une cavité ménagée dans le fond de la capsule et dans laquelle débouche le cathéter.

Cette capsule est implantée sous la peau, dans une zone facilement accessible, par exemple au niveau de la poitrine du patient.

Pour réaliser une injection d'un produit actif liquide, il suffit au praticien, après avoir localisé la capsule, d'introduire l'aiguille d'une seringue à travers la peau du patient, à l'intérieur de la capsule après traversée de la pastille.

Il est alors procédé à l'injection de produits dans la cavité ménagée dans la capsule, le produit passant ensuite par l'intermédiaire du cathéter dans le vaisseau ou autre organe à perfuser. Après retrait de l'aiguille, il est procédé à l'injection d'un produit de rinçage, afin d'éviter qu'une certaine quantité de principe actif demeure dans la capsule et le cathéter.

Il faut noter que, de par ses propriétés, la pastille assure l'étanchéité de la capsule vis-à-vis de l'extérieur après retrait de l'aiguille de la seringue.

Avantageusement, la capsule possède une forme générale circulaire, et le couvercle une forme générale tronconique, sans présenter aucun angle vif, la pastille étant disposée dans la partie centrale du couvercle au niveau de l'ouverture de celui-ci.

La forme de cette capsule évite les contacts blessants et irritants avec les tissus, qui nécessiteraient un retrait de la capsule après une certaine durée d'implantation. En ce qui concerne la forme conique du couvercle, elle permet le repérage de la capsule sous la peau ainsi que la localisation de la pastille de celle-ci à travers laquelle doit être réalisée l'injection.

Afin de faciliter encore la localisation de la pastille, le bord d'appui du couvercle sur la pastille, situé en périphérie de l'ouverture du couvercle, est incliné de l'extérieur vers l'intérieur et de la face extérieure du couvercle vers le fond de la capsule.

Selon une autre caractéristique de l'invention, le fond de la capsule possède un rebord périphérique dans lequel sont ménagés un certain nombre de trous permettant la suture de la capsule sur les tissus. Il est en effet important de réaliser une bonne tenue de la capsule à l'intérieur du corps pour résister notamment à la pression exercée lors de l'enfoncement de l'aiguille à travers la pastille.

Le fond et le couvercle de la capsule sont assemblés par collage, l'un au moins de ces deux éléments présentant une gorge dans la zone d'assemblage pour assurer une excellente solidarisation par augmentation de l'épaisseur de colle.

En ce qui concerne la cavité ménagée dans le fond de la capsule, elle est délimitée par une partie en forme de cheminée dont le bord libre sert de siège à la pastille, le couvercle présentant, pour sa part, plusieurs nervures radiales venant au contact de la périphérie de la cheminée. Ces nervures assurent, outre le renforcement du couvercle, le centrage de celui-ci par rapport au fond de la capsule, ainsi que le positionnement de la pastille par rapport à ces deux éléments.

Selon une autre caractéristique, le fond présente un canal débouchant radialement dans sa cavité, de diamètre intérieur égal au diamètre extérieur du cathéter, et comportant une rainure annulaire permettant l'injection de colle contre la paroi périphérique du cathéter.

Afin d'assurer une bonne protection du cathéter lors des mouvements que peut subir la capsule, et lors de l'implantation de celle-ci, un bossage faisant saillie du rebord périphérique du fond est prévu dans la zone dans laquelle débouche le canal servant au passage du cathéter.

De toute façon, l'invention sera bien comprise à l'aide de la description qui suit, en référence au dessin schématique annexé représentant, à titre d'exemple non limitatif, une forme d'exécution de cette capsule :

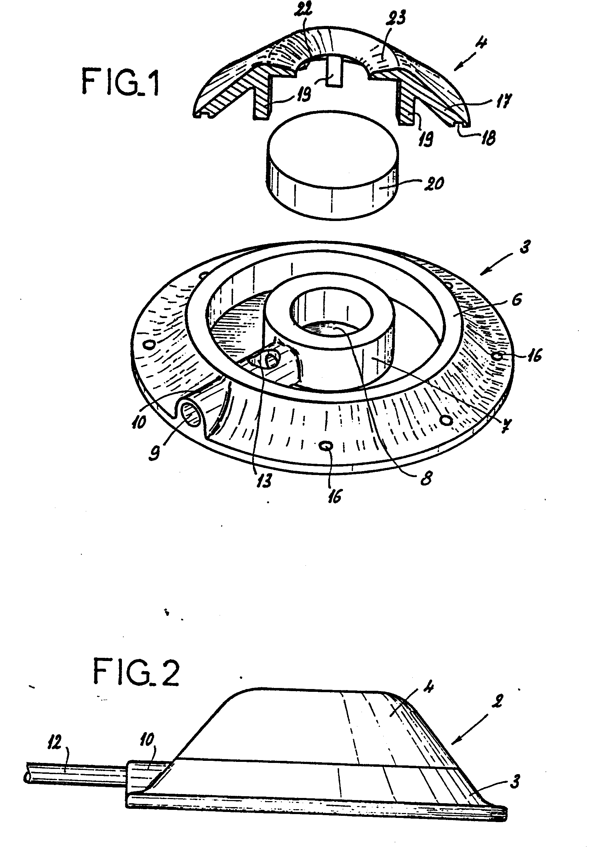

Figure 1 en est une vue en perspective éclatée, avec coupe partielle du couvercle ;

Figure 2 en est une vue de côté ;

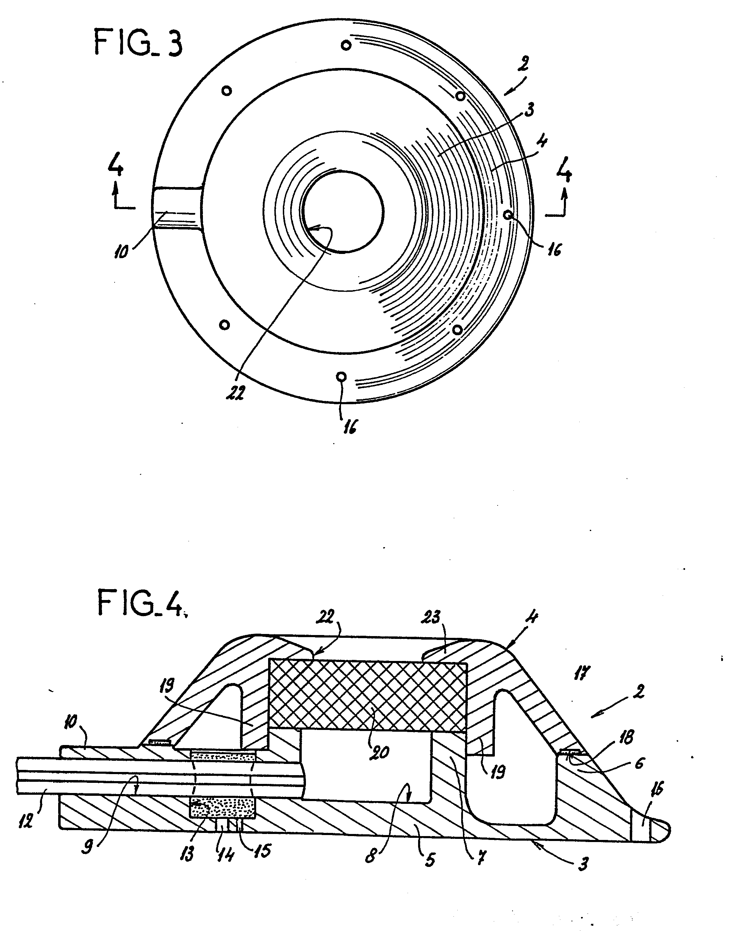

Figure 3 en est une vue de dessus ;

Figure 4 en est une vue en coupe longitudinale selon la ligne 4-4 de figure 3.

La capsule de perfusion (2), représentée au dessin, possède une forme générale circulaire d'environ 37 millimètres de diamètre, et une hauteur de l'ordre de 12 millimètres.

Cette capsule comporte un fond (3) et un couvercle (4) réalisés tous deux en une matière rigide et biocompatible, telle que résine époxy ou polyuréthane. Le fond présente une surface (5) de laquelle fait saillie, d'une part, un bord périphérique (6) et, d'autre part, une cheminée centrale (7) délimitant une cavité (8).

A l'intérieur de la cavité (8) débouche un canal radial (9), ménagé au niveau de la partie extérieure du fond (3) dans un bossage (10). Le canal (9) sert au montage de l'extrémité d'un cathéter (12) d'une longueur de l'ordre de 80 centimètres et dont les diamètres extérieur et intérieur sont, respectivement, de 2,2 et 0,5 millimètres.

La fixation du cathéter (12) dans le canal (9) est réalisée par injection de colle dans une rainure (13) débouchant dans la face interne du canal (9). Après mise en place du cathéter (12), la colle est injectée par un orifice (14) débouchant dans la rainure (13), l'air s'échappant par un évent (15) débouchant également dans cette dernière.

La forme de cette rainure est arrondie, de manière à éviter de piéger des bulles d'air à l'intérieur de celle-ci. Après remplissage de colle, les trous d'injection et d'évent, respectivement (14) et (15), sont bouchés. Ce collage assure l'étanchéité, d'une part, entre l'exté-

4

rieur du cathéter et l'intérieur de la capsule et, d'autre part, entre la périphérie de l'appareil et la cavité (8).

Le fond (3) présente un rebord périphérique dans lequel sont ménagés des trous (16), au nombre de sept dans la forme d'exécution représentée au dessin, permettant la fixation de la capsule par des sutures après son implantation sous la peau.

Le couvercle (4) présente, pour sa part, un bord périphérique (17) destiné à venir en appui contre le bord (6) du fond (3). Dans le bord (17) est ménagée une rainure (18) permettant d'augmenter l'épaisseur de colle lors de la fermeture de la capsule par collage.

Dans la forme d'exécution représentée au dessin, la capsule (4) comporte quatre nervures (19) faisant saillie vers l'intérieur, et destinées à prendre appui à la périphérie de la cheminée (7). Ces nervures (19) assurent, outre la rigidification du couvercle, le centrage de celui-ci et d'une pastille (20) réalisée en un matériau auto-obturant, tel que du silicone de faible dureté. La face supérieure de la capsule (4) comporte une ouverture centrale (22) délimitée par un rebord (23) prenant appui sur la pastille (20) et assurant la compression de celle-ci. Ce rebord (23) présente une face extérieure inclinée de l'extérieur vers l'intérieur, et de haut en bas.

D'un point de vue pratique, cette capsule étant implantée sous la peau du patient, dans une zone du corps de celui-ci très accessible, telle que la poitrine, et le cathéter débouchant dans une veine ou autre organe à perfuser, il suffit, pour réaliser une injection, de localiser la capsule (2) et plus précisément la pastille (20), ce qui est facilité compte tenu de la forme de la capsule. Après localisation, l'opérateur introduit l'aiguille d'une seringue à travers la peau du patient et la pastille auto-obturante avant de réaliser l'injection dans la cavité (8) et dans le cathéter (12).

Comme il ressort de ce qui précède, l'invention apporte une grande amélioration à la technique existante en fournissant un dispositif de conception simple permettant de réaliser des injections fréquentes sans aucun dommage pour les vaisseaux, comme tel est le cas actuellement.

## REVENDICATIONS

1. - Dispositif de perfusion, caractérisé en ce qu'il est constitué par une capsule (2) réalisée en un matériau biocompatible, destinée à être implantée sous la peau du patient, comprenant un fond (3) dans lequel débouche une extrémité d'un cathéter (12), dont l'autre extrémité débouche dans une veine ou autre organe à perfuser, et un couvercle (4) qui, fixé sur le fond et tourné du côté de la peau du patient, présente une ouverture centrale (22) et sert au maintien en compression contre le fond d'une pastille auto-obturante (20), de part et d'autre de laquelle sont situées, d'une part, l'ouverture (22) ménagée dans le couvercle et, d'autre part, une cavité (8) ménagée dans le fond de la capsule et dans laquelle débouche le cathéter (12).

2. - Dispositif selon la revendication 1, caractérisé en ce que la capsule possède une forme générale circulaire, et le couvercle (4) une forme générale tronconique, sans présenter aucun angle vif, la pastille (20) étant disposée dans la partie centrale du couvercle (4) au niveau de l'ouverture (22) de celui-ci.

3. - Dispositif selon l'une quelconque des revendications 1 et 2, caractérisé en ce que le bord d'appui (23) du couvercle (4) sur la pastille (20), situé en périphérie de l'ouverture (22) du couvercle, est incliné de l'extérieur vers l'intérieur et de la face extérieure du couvercle vers le fond de la capsule.

4. - Dispositif selon l'une quelconque des revendications 1 à 3, caractérisé en ce que le fond (3) de la capsule possède un rebord périphérique dans lequel sont ménagés un certain nombre de trous (16) permettant la suture de la capsule sur les tissus.

5. - Dispositif selon l'une quelconque des revendications 1 à 4, caractérisé en ce que le fond (3) et le couvercle (4) de la capsule sont assemblés par collage, l'un au moins de ces deux éléments présentant une gorge (18), dans la zone d'assemblage, pour assurer une excellente solidarisation par augmentation de l'épaisseur de colle.

6. - Dispositif selon l'une quelconque des revendications 1 à 5, caractérisé en ce que la cavité (8), ménagée dans le fond de la capsule, est délimitée par une partie (7) en forme de cheminée dont le bord libre sert de siège à la pastille, le couvercle présentant, pour sa part, plusieurs nervures radiales (19) venant au contact de la périphérie de la cheminée.

6

7. - Dispositif selon l'une quelconque des revendications 1 à 6, caractérisé en ce que le fond (3) présente un canal (9) débouchant radialement dans sa cavité, de diamètre intérieur égal au diamètre extérieur du cathéter (12), et comportant une rainure (13) annulaire permettant l'injection de colle contre la paroi périphérique du cathéter.

FIG_1

FIG_2

FIG_3

FIG_4.

Office européen
des brevets

# RAPPORT DE RECHERCHE EUROPEENNE

EP 84 42 0147

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| Y | FR-A-2 306 712 (METAL BELLONS COMPANY) <br> * Figures 1,2; page 5, lignes 5-34 * | 1-3 | A 61 M 5/00 |
| Y | US-A-3 310 051 (R. SCHULTE) <br> * Figures et texte * | 1-3 | |
| A | MEDICAL PROGRESS THROUGH TECHNOLOGY, vol. 9, no. 1, 1982, pages 17-25, Berlin, DE; G.A. CARLSON et al.: "An implantable, remotely programmable insulin infusion system" <br> * Page 21, colonne de droite, paragraphe: "Reservoir, tubing and catheter"; figures 2,4 * | 1,2,4, 5 | |
| A | US-A-3 344 785 (D. HAMILTON) <br> * Colonne 2, lignes 54-69; figures 5,6 * | 3 | **DOMAINES TECHNIQUES RECHERCHES (Int. Cl. ³)** <br><br> A 61 M <br> A 61 F |
| A | DE-A-3 102 993 (P. BOTTERMAN) <br> * Figures et texte * | 1 | |

Le présent rapport de recherche a été établi pour toutes les revendications

| Lieu de la recherche <br> LA HAYE | Date d'achèvement de la recherche <br> 17-10-1984 | Examinateur <br> VEREECKE A. |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82